# EUROPÄISCHE PATENTSCHRIFT

(11) **EP 4 097 426 B1**
(45) Veröffentlichungstag und Bekanntmachung des Hinweises auf die Patenterteilung: **18.12.2024**
(21) Anmeldenummer: 21702268.0
(22) Anmeldetag: 28.01.2021
(51) Int. Cl.: G01F 1/58, A61M 1/16, A61M 1/36, G01F 15/02

(54) **FLUSSSENSOR UND VERFAHREN ZUM MESSEN EINES FLUSSES**
FLOW SENSOR AND METHOD FOR MEASURING A FLOW
CAPTEUR DE DÉBIT ET PROCÉDÉ POUR MESURER UN DÉBIT

(30) Priorität: 31.01.2020 DE 102020102485
(43) Veröffentlichungstag der Anmeldung: 07.12.2022
(73) Patentinhaber: Fresenius Medical Care Deutschland GmbH, 61352 Bad Homburg (DE)
(72) Erfinder: NIKOLIC, Dejan, 65812 Bad Soden (DE); HEIDE, Alexander, 65817 Eppstein (DE)
(74) Vertreter: Herrmann, Uwe
(86) Internationale Anmeldenummer: PCT/EP2021/052027
(87) Internationale Veröffentlichungsnummer: WO 2021/152039

(56) Entgegenhaltungen:
- DE-A1- 102010 003 642
- US-A1- 2015 135 848

## Beschreibung

Die vorliegende Erfindung betrifft einen Flusssensor zur Messung einer Flussdifferenz sowie ein Verfahren zum Messen einer Flussdifferenz.

Insbesondere betrifft die vorliegende Erfindung einen magnetisch induktiven Sensor zur Messung der Differenz eines Flusses von Fluidströmen, die durch zwei Messkammern bzw. Kanäle fließen.

Die Flussdifferenz (Eingang minus Ausgang) (auch als Flussbilanz bezeichnet) von Fluiden bzw. Flüssigkeiten ist eine wichtige Größe beispielsweise bei Dialysebehandlungen. Es ist wichtig, dass der Wasserhaushalt von Dialysepatienten im Rahmen einer Behandlung nicht negativ beeinflusst wird, sondern das Erreichen eines gesunden Wasserhaushalts durch die Behandlung unterstützt wird. Dazu muss die Flüssigkeitsbilanz eines Patienten möglichst genau bekannt sein.

Grundsätzlich kann die Flussdifferenz direkt gemessen oder durch die Verrechnung zweier Absolutmessungen des Flusses ermittelt werden. Eine direkt differentielle Flussmessung ist genauer (oder kostengünstiger) erreichbar als eine indirekte Differenzmessung durch eine Kombination zweier Absolut-Messungen, deren Differenz berechnet wird, denn Absolut-Messungen sind per se sehr aufwändig bezüglich ihrer Messgenauigkeit und zudem fehleranfälliger. Eine direkt differentielle Messung kann dagegen mit geringerem Aufwand eine höhere Genauigkeit erreichen, weil die (pro Kanal) auftretenden Messfehler der Absolut- Messung sich gegenseitig aufheben. Dennoch ist die Messgenauigkeit weiterhin ein Problem bei bekannten differentiellen Flusssensoren, wie z.B. bei dem aus der JP5467576 (B2) bekannten Gattungsgemäßen Flusssensor bekannt.

Ausgehend von diesem wurden vorteilhafte Weiterbildungen beispielsweise in der WO 2015/197424 und der EP 3 254 066 B1 offenbart, aus welchen jeweils Sensor-Einwegkassetten mit Elektroden zur Anordnung in einem Magnetjoch bekannt sind.

Das Messprinzip besteht jeweils darin, zwei Messkanäle vorzusehen, die jeweils senkrecht zur Fließrichtung von einem Magnetfeld durchdrungen werden und zwei oder mehr (zu Magnetfeld und Fließrichtung senkrecht angeordnete) Elektroden aufweisen. Durch das Magnetfeld werden im Fluid ungleichnamige Ladungsträger getrennt (Hall-Effekt) und senkrecht zum Magnetfeldvektor und der Fließrichtung transportiert.

Im Fall der in der WO 2015/197424 und der EP 3 254 066 B1 offenbarten Lösungen befinden sich an den beiden Kanalrändern Elektroden, die zur Messung des Potentials, das aus der Ladungstrennung resultiert, gedacht sind. Das aus der Ladungstrennung resultierende elektrische Potential in der Flüssigkeit wird mittels dieser Messelektroden gemessen. Weiteren Stand der Technik stellen die Dokumente DE102010003642A1 und US2015/135848A1 dar.

Bei diesen bekannten Sensoren besteht jedoch das Problem, dass eine für eine extrakorporale Blutbehandlung, insbesondere eine Dialyse, ausreichende Messgenauigkeit über die Zeit nicht gewährleistet wird.

Dies rühr daher her, dass über die Zeit bereits mikroskopische Verrückungen oder Erschütterungen der Sensorkassette ausreichen, um zu nicht-tolerierbaren Messfehlern zu führen: Da das Magnetfeld bei den bekannten Sensoren nicht perfekt homogen ist, werden die beiden Messkammern bzw. Kanäle unterschiedlich von Feldlinien eines Magnetfelds durchdrungen.

Temperaturänderungen und Vibrationen reichen bei den bekannten Sensoren daher bereits aus, um die Messgenauigkeit aus dem für Dialyse tolerierbaren Bereich herauszuführen. Vibrationen treten beispielsweise durch Pumpen, z.B. Rollenpumpen, Membranpumpen, durch die Eigenvibration oder durch Druckpulse auf das durch die Messkanäle fließende Fluid auf. Im Fall der Dialyse kann zudem die Bewegung der Leitungen durch einen Patienten zu weiteren Verrückungen des Sensors führen und somit die Messgenauigkeit weiter verschlechtern.

Der vorliegenden Erfindung liegt die Aufgabe zugrunde, die aus dem Stand der Technik bekannten Probleme abzumildern oder gar ganz zu beseitigen und insbesondere einen verbesserten differentiellen Flusssensor bereitzustellen, der über eine Messdauer von mehreren Stunden eine ausreichende Messgenauigkeit bietet. Diese Aufgabe wird durch einen Flusssensor sowie ein Blutbehandlungsgerät und ein System gemäß den unabhängigen Ansprüchen gelöst. Weitere vorteilhafte Weiterbildungen der Erfindung sind Gegenstand der Unteransprüche.

Ein erfindu8nsggemäßer Flusssensor zum Messen einer Flussdifferenz weist auf: mindestens zwei Messkammern, durch welche Fluid leitbar ist; mindestens ein Mittel zur Erzeugung eines Magnetfelds zur Ladungstrennung in einem die mindestens zwei Messkammern durchströmenden Fluid, und mindestens ein Mittel zur Messung eines elektrischen Potentials in dem die mindestens zwei Messkammern durchströmenden Fluid, wobei die mindestens zwei Messkammern derart angeordnet sind, dass sie von derselben Magnetfeldlinie des Magnetfelds zur Ladungstrennung durchlaufen werden. Zusätzlich aufweist der Flusssensor einen Temperatursensor zur Messung der Temperatur eines Fluids.

In anderen Worten ist gemäß der Erfindung bei einem Flusssensor die Anordnung der Messkammern (bzw. Kanäle) derart, dass die geometrischen Bereiche beider Messkanäle, in denen die Flüsse des Fluids gemessen werden, derart angeordnet sind, dass sie von denselben Magnetfeldlinien durchdrungen werden.

Die beiden Messkammern bzw. die Bereiche der Messkammern sind beispielsweise übereinander, untereinander oder auch nebeneinander angeordnet. Die Anordnung ist beliebig, solange sichergestellt ist, dass beiden Messkammern bzw. die Bereiche der Messkammern, in denen die Flüsse des Fluids gemessen werden, von denselben Magnetfeldlinien durchlaufen werden.

Ein Flusssensor mit einer solchen Anordnung ist robust gegen Temperaturänderungen, Vibrationen und weitere Umwelteinflüsse, weil beide Messkammern bzw. Kanäle stets im gleichen Maß von den Umwelteinflüssen betroffen sind.

Daraus ergibt sich, dass bei einer differentielle Messung die Änderungen nicht ins Gewicht fallen, da in beiden Messkammern dieselben Bedingungen bezüglich des Magnetfelds herrschen bzw. das Magnetfeld in beiden Messkammern in gleichem Maße von den Umwelteinflüssen beeinflusst wird.

Das Mittel zur Messung eines elektrischen Potentials umfasst vorzugsweise eine oder mehrere Messelektroden. Vorzugsweise weist jedes Messkammer bzw. jeder Kanal jeweils zwei Messelektroden auf.

In anderen Worten ist gemäß einer bevorzugten Ausführungsform jeder der zwei Messkammern ein Mittel zur Messung eines elektrischen Potentials zugeordnet und die mindestens zwei Messkammern und / oder die diesen zugeordneten Mittel zur Messung eines elektrischen Potentials fluchten miteinander.

In einer besonders bevorzugten Ausführung sind die Messelektroden beider Kanäle in einer Flucht bzw. derart fluchtend angeordnet, dass ihre Projektion auf eine (parallel zum Fluss des Fluids und senkrecht zum Magnetfeld verlaufende) Ebene exakt deckungsgleich ist.

Dadurch ist der Flusssensor bzw. die diesen umfassende Kassette so ausgeführt, dass sie in einem Magnetjoch, beispielsweise eines Blutbehandlungsgeräts, so eingelegt werden kann, dass die Messbereiche in beiden Kanälen von exakt denselben Feldlinien durchdrungen werden.

Auf welche Weise das aus der magnetisch- induzierten Ladungstrennung resultierende elektrische Potential gemessen wird, ist beliebig. Beispielsweise können direkt im Kontakt zu dem Fluid stehende Elektroden verwendet werden oder es kann indirekt kapazitiv gemessen wird. Weiterhin ist die Form, Anzahl und Positionierung der Messmittel, insbesondere der Messelektroden, beliebig. Es können auch mehr als zwei Elektroden eingesetzt werden.

Ebenso wäre eine indirekte Messung des Potentials denkbar: Hierbei wird beispielsweise kapazitiv das elektrische Feld gemessen. Bei einer kapazitiven Messung befinden sich die Messmittel zur Messung des elektrischen Feldes nicht in dem Fluid, sondern sind (durch einen Isolator getrennt) außerhalb davon angeordnet. Vorzugsweise ist ein einfindungsgemäßer Flusssensor ein magnetisch-induktiver differentieller Durchflusssensor.

Es ergeben sich zwei synergetische Vorteile, wenn auch eine Temperatur im Fluss-sensor gemessen wird: Erstens ist in der Regel für die Ausführung einer extrakorporalen Blutbehandlungen eine Temperaturmessung der Fluide bzw. Flüssigkeiten erforderlich. Wenn der Flusssensor integriert die Temperaturmessung mitübernimmt, kann vorteilhaft ein zusätzlicher Sensor, der zusätzliches Flüssigkeitsvolumen aufnehmen würde, eingespart werden.

Zweitens kann mit Kenntnis der Temperatur eine Korrektur von Flussmesswerten oder der Messgenauigkeit vorgenommen werden.

Vorzugsweise kommt ein Flusssensor, der auch als Temperatursensor dient, im Zusammenspiel mit einem extrakorporalen Blutbehandlungsgerät, insbesondere einer Dialysemaschine, zum Einsatz.

Gemäß einer bevorzugten Ausführungsform weist eine Behandlungsmaschine, in die die Kassette mit dem erfindungsgemäßen Flusssensor eingelegt werden kann, eine Infrarot-Diode und eine Auswertungseinrichtung auf, die das von der Diode gemessene Licht auswertet und so die Temperatur eines Mediums bzw. Fluids, was sich vor der Diode befindet, ermittelt. Korrespondierend zu der Diode ist eine Aussparung bzw. eine Art "Fenster" in der Kassette des Flusssensors vorgesehen.

Die Wanddicke im Fensterbereich beträgt vorzugsweise ca. 300µm- ca. 600µm, insbesondere ca. 500 µm. Andere Dicken sind denkbar. Alternativ kann beispielsweise ein PT1000-Sensor zur Temperaturmessung eingesetzt werden.

Der Temperatursensor ist vorzugsweise in einer den Flusssensor aufweisenden Kassette integriert und / oder fest verbaut. In diesem Fall ist vorzugsweise auch eine zugehörige Auswertungseinrichtung in der Kassette integriert. Alternativ oder zusätzlich kann der Temperatursensor und / oder die Auswertungseinrichtung auch seitens des Blutbehandlungsgeräts vorgesehen sein und der Kassette zugeordnet bzw. zugeschaltet sein.

Alternativ oder zusätzlich kann der Flusssensor einen Leitfähigkeitssensor zur Messung der Leitfähigkeit eines Fluids aufweisen. Auch der Leitfähigkeitssensor ist vorzugsweise in einer den Flusssensor aufweisenden Kassette integriert und / oder fest verbaut. In diesem Fall ist vorzugsweise auch eine zugehörige Auswertungseinrichtung in der Kassette integriert.

Alternativ oder zusätzlich kann der Temperatursensor und / oder die Auswertungseinrichtung auch seitens des Blutbehandlungsgeräts vorgesehen sein und der Kassette zugeordnet bzw. zugeschaltet sein.

Eine bevorzugte Ausführungsform für eine Messanordnung der Leitfähigkeit weist drei Elektroden auf, die in Flussrichtung hintereinander im Fluidkanal (in Kontakt mit dem Fluid) angeordnet sind. Dabei ist die mittlere Elektrode vorzugsweise als flächiges Rechteck ausgeführt und die beiden anderen Elektroden jeweils als den Fluidkanal umgreifendes "U". Alle drei Elektroden sind bevorzugt aus rostfreiem Stahl gefertigt.

Im Messbetrieb wird eine Spannung so an die mittlere Elektrode angelegt, dass ein Strom durch das Fluid zu den stromabwärts und stromaufwärts gelegenen Elektroden fließt. Die beiden äußeren Elektroden sind geerdet. Der Spannungsabfall zwischen der mittleren Elektrode und den äußeren Elektroden wird gemessen, ebenso der fließende Strom. Aus Strom und Spannungsabfall wird der Leitwert gemessen. Bei Kenntnis der Geometrie lässt sich daraus die Leitfähigkeit des Fluids bestimmen.

Die Auswertungsvorrichtung, die aus den elektrischen Messwerten an den Elektroden eine Leitfähigkeit bestimmt, ist vorzugsweise Teil der Behandlungsmaschine, nicht der Fluss-Sensor-Kassette. So kann die Sensor-Kassette besonders preisgünstig ausgestaltet werden, was eine Ausgestaltung als Wegwerfartikel (Disposable) ermöglicht.

Grundsätzlich kann der Flusssensor und / oder die diesen aufweisende Kassette als Disposable oder als Mehrfachartikel ausgestaltet sein.

Ein weiterer Aspekt der vorliegenden Erfindung betrifft ein Blutbehandlungsgerät, insbesondere ein Dialysegerät, welches dazu ausgelegt ist, mit einem erfindungsgemäßen Flusssensor eingesetzt zu werden oder welches einen erfindungsgemäßen Flusssensor aufweist.

Es hat sich in der Praxis als vorteilhaft erwiesen, wenn das Blutbehandlungsgerät dazu ausgelegt ist, einen erfindungsgemäßen Flusssensor zumindest teilweise lösbar aufzunehmen und vorzugsweise zu diesem Zweck mit einer Aufnahme für den Flusssensor ausgestattet ist.

Die Ausgestaltung der Aufnahme ist beliebig. Beispielsweise kann die Aufnahme des Blutbehandlungsgeräts wie eine Zange (vorzugsweise mit innenliegenden elektrischen Kontakten) oder als Vertiefung, wie z.B. als Schlitz ausgeführt sein.

Ebenso ist es denkbar, dass die Aufnahme die Gestalt einer Vertiefung hat, die mittels einer Tür verschlossen werden kann, wenn die Sensor-Kassette in die Vertiefung eingelegt ist. Die Tür kann so ausgestaltet, z.B. vorgespannt sein, dass sie die Vertiefung verschlossen hält, wenn nicht ein Benutzer aktiv die Tür öffnet.

Ebenso können Verriegelungselemente vorgesehen sein, welche die Sensor-Kassette an oder in dem Blutbehandlungsgerät halten.

Weiterhin hat es sich als vorteilhaft erwiesen, wenn das mindestens eine Mittel zur Erzeugung eines Magnetfelds zur Ladungstrennung in einem die mindestens zwei Messkammern durchströmenden Fluid seitens des Blutbehandlungsgeräts anordnet ist. In anderen Worten sind vorzugsweise einer oder mehrere Magneten an dem Blutbehandlungsgerät vorgesehen.

Vorzugsweise sind das mindestens eine Mittel zur Erzeugung eines Magnetfelds zur Ladungstrennung mindestens zwei Magnetpole, welche auf einander gegenüberliegenden Seiten einer Aufnahme für den Flusssensor bzw. die Sensor-Kassette angeordnet. Insbesondere, wenn der Flusssensor als Disposable ausgestaltet ist, ist diese Ausführungsform vorteilhaft. Alternativ können auch zwei Elektromagnete eingesetzt werden.

Ein weitere Aspekt der Erfindung betrifft die Verwendung eines erfindungsgemäßen Flusssensors und / oder eines erfindungsgemäßen Blutbehandlungsgeräts zur Blut-Blut-Bilanzierung. Durch die kompakte Bauweise des erfindungsgemäßen Flusssensors wird nur wenig Blut für die Messung benötigt, weswegen der erfindungsgemäße Flusssensor für eine derartige Anwendung besonders geeignet ist.

Beispielsweise offenbart die WO 2019/139671 A1 eine Behandlung, welche eine "Spende" der Dialysefähigkeit einer gesunden Niere einer ersten Person an eine zweite Person mit Niereninsuffizienz umfasst. Hierbei kann auch ein Dialysator zum Einsatz kommen. Ein erfindungsgemäßer Bilanzsensor kann verschiedentlich eingesetzt werden, um die Änderung des Flüssigkeitshaushalts (Wasserbilanz) der Person mit Niereninsuffizienz zu messen.

Beispielsweise kann ein erfindungsgemäßer Flusssensor zur Erfassung der Fluidbilanz (Eingang minus Ausgang) des Dialysators für die niereninsuffiziente Person eingesetzt werden. Hierbei wird angenommen, dass die gesunde Person ihren Wasserhaushalt selbstständig regulieren kann.

Optional könnte ein zweiter Bilanzsensor zur Vervollständigung zum Einsatz kommen, der für die gesunde Person die Fluidbilanz (Eingang minus Ausgang) des Dialysators ermittelt.

Alternativ können auch zwei erfindungsgemäße Flusssensoren zur Blut-Blut-Bilanzierung eingesetzt werden. Jeweils am Eingang und am Ausgang des Dialysators wird hierbei vorzugsweise ein erfindungsgemäßer differentieller Flusssensor eingesetzt. In dieser Ausführungsform einer Verwendung eines hier vorgestellten Flusssensors würde die Flüssigkeitsbilanz für die beiden Dialysatorhälften bzw. für die beiden Personen also in einem zweiten Schritt erfolgen: Im ersten Schritt wird eine "Eingangsbilanz" (Eingangsfluss Person 1 in den Dialysator wird differentiell gegenüber dem Eingangsfluss von Person 2 in den Dialysator gemessen) von einem ersten Sensor gemessen und gleichzeitig eine "Ausgangsbilanz" am Ausgang des Dialysators gemessen. Aus diesen beiden Bilanzen kann in einem zweiten Schritt eine Fluidbilanz für beide Personen berechnet werden, beispielsweise indem die "Ausgangsbilanz" von der "Eingangsbilanz" abgezogen wird.

Mittels des Flusssensors am Eingang des Dialysators wird die Differenz des Flusses am Eingang des Dialysators für die gesunde Person und die Person mit Niereninsuffizienz ermittelt.

Analog dazu wird am Ausgang des Dialysators die Differenz des Flusses aus dem Dialysator für die gesunde Person und die Person mit Niereninsuffizienz ermittelt.

Über beide Differenzen kann man auf den Netto-Wasser-Fluss beider Personen schließen.

Ein weiterer Aspekt der Erfindung betrifft ein Verfahren zur Messung einer Flussdifferenz eines Fluids, vorzugsweise mit einem Flusssensor gemäß der vorliegenden Erfindung, mit den Schritten:
Leiten von Fluid durch mindestens zwei Messkammern bzw. Kanäle,
Erzeugen eines Magnetfelds zur Ladungstrennung in dem Fluid,
Messen eines elektrischen Potentials in dem die mindestens zwei Messkammern durchströmenden Fluid, wobei
das Magnetfeld zur Ladungstrennung derart erzeugt wird, dass dieselbe Magnetfeldlinie des Magnetfelds zur Ladungstrennung die mindestens zwei Messkammern
durchläuft, und

Erfassen der Temperatur des Fluids, vorzugsweise mittels eines in den Flusssensor integrierten Temperatursensors.

Vorzugsweise umfasst das Verfahren auch den vorgeschalteten Schritt des Einlegens einer Flusssensor-Kassette in eine entsprechende Aufnahme eines erfindungsgemäßen Blutbehandlungsgeräts.

Alternativ oder zusätzlich umfasst das Verfahren zudem den Schritt:
Erfassen der Leitfähigkeit des Fluids, vorzugsweise mittels eines in den Flusssensor integrierten Leitfähigkeitssensors.

Weitere Vorteile, Merkmale und Effekte der vorliegenden Erfindung ergeben sich aus der nachstehenden Beschreibung bevorzugter Ausführungsformen unter Bezugnahme auf die Figuren. Gleiche oder ähnliche Bauteile sind in den Zeichnungen mit denselben Bezugszeichen bezeichnet. Hierbei zeigt:
Fig. 1 einen Querschnitt eines erfindungsgemäßen Flusssensors;
Fig. 2 einen weiteren Querschnitt eines erfindungsgemäßen Flusssensors;
Fig. 3 einen Längsschnitt eines erfindungsgemäßen Flusssensors; und
Fig. 4 ein Schaubild, welches den Einsatz eines erfindungsgemäßen Sensors zur Blut-Blut-Bilanzierung verdeutlicht.

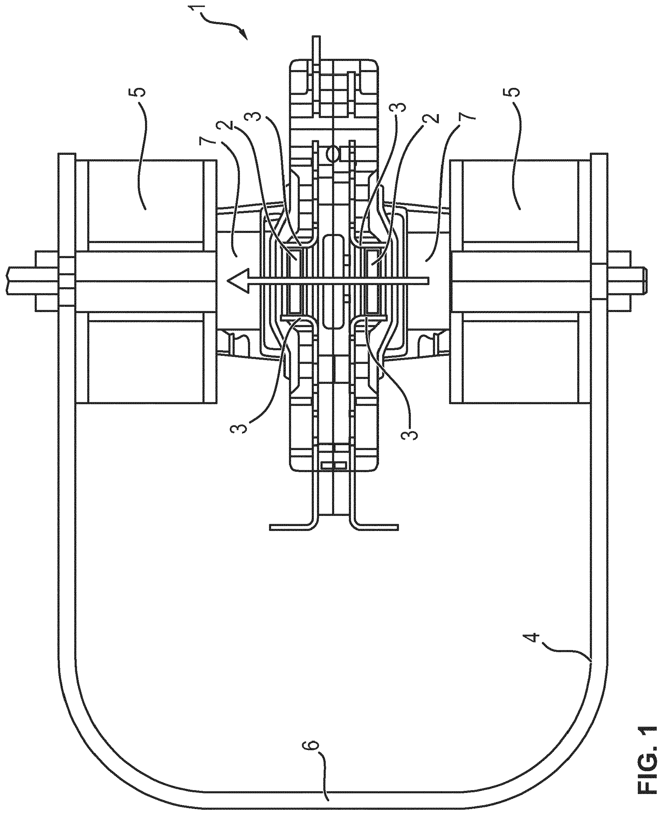

Wie in Fig. 1 gezeigt, weist ein erfindungsgemäßer Flusssensor 1 zwei Messkammern bzw. Kanäle 2 auf, welche bei der Messung von einem Fluid durchströmt werden. Auf einander gegenüberliegenden Seiten einer jeden Messkammer 2 ist jeweils eine Messelektrode 3 angeordnet, um ein aufgrund der Ladungstrennung in dem Fluid entstandenes elektrisches Potential zu messen.

Der Sensor ist in ein Magnetjoch 4 eingesetzt, dass zwei Magnetspulen 5 aufweist, welche mittels eines Bügels 6 miteinander verbunden sind. Magnetpole 7 sind zwischen den Spulen 5 und den Messkammern 2 angeordnet. Mittels der Magnete wird ein Magnetfeld erzeugt, dessen Magnetfeldvektor durch den in Fig. 1 gezeigten senkrechten Pfeil wiedergegeben wird. Der Magnetfeldvektor verläuft orthogonal zur Flussrichtung des Fluids in den Messkammern, welche in der Darstellung in Fig. 1 orthogonal zur Papierebene verläuft.

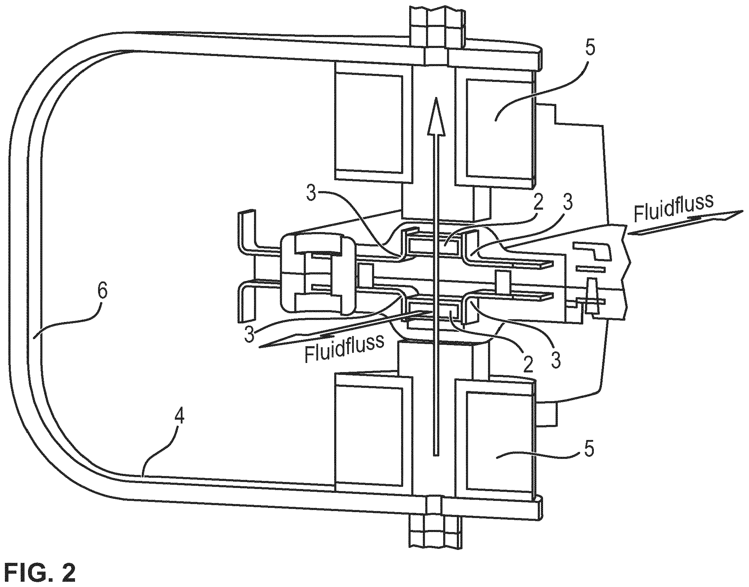

In Fig. 2 ist die Flussrichtung des Fluids durch zwei gegenläufige Pfeile widergegeben. Der Magnetfeldvektor verläuft orthogonal zur Flussrichtung des Fluids.

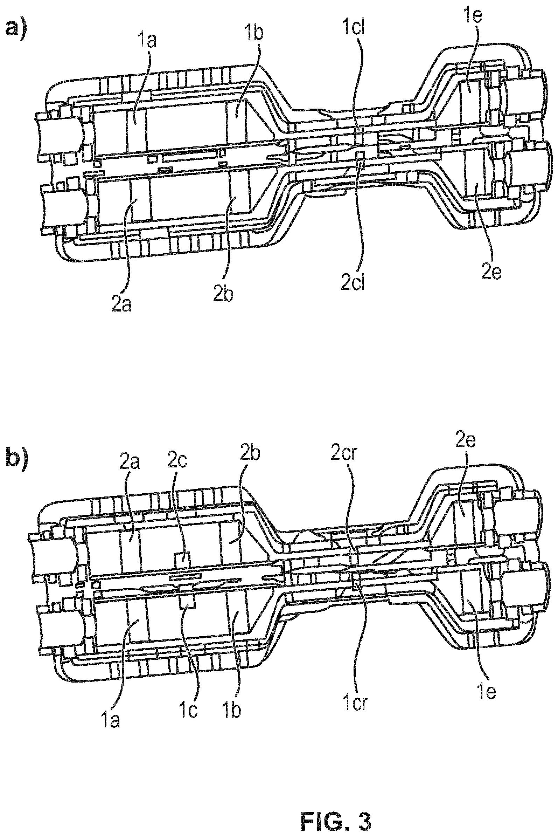

Fig. 3 zeigt in den Abbildungen 3a) und 3b) jeweils einen Längsschnitt eines erfindungsgemäßen Flusssensors. Die Darstellung in Abbildung 3b) ist gegenüber der Darstellung in Abbildung 3a) gedreht.

Der in Fig. 3 gezeigte Flusssensor ist nicht nur zur differentiellen Flussmessung ausgelegt, sondern weist auch einen integrierten Temperatursensor und einen integrierten Leitfähigkeitssensor auf.

Wie in den Abbildungen 3a und 3b gezeigt, weist ein Flusssensor gemäß dieser Ausführungsform drei Elektroden zur Leitfähigkeitsmessung auf. Diese sind mit den Bezugszeichen 1a, 1b, 1c bzw. 2a,2b,2c bezeichnet. Die mit dem Bezugszeichen 1a bzw. 2a bezeichnete Elektrode dient im Rahmen der Leitfähigkeitsmessung als Masse. Die mit dem Bezugszeichen 1c bzw. 2c bezeichnete Elektrode dient im Rahmen der Leitfähigkeitsmessung als heiße Elektrode. Die mit dem Bezugszeichen 1b bzw. 2b bezeichnete Elektrode kommt ebenfalls im Rahmen der Leitfähigkeitsmessung zum Einsatz.

Die Elektroden 1cl und 1cr dienen vorzugsweise als Messelektroden der Flussmessung und sind jeweils links (1cl) und rechts (1cr) einer Messkammer angeordnet. Die Elektroden 2cl und 2cr dienen vorzugsweise als Messelektroden der Flussmessung und sind jeweils links (2cl) und rechts (2cr) einer Messkammer angeordnet.

Die Bezugszeichen 1cl und 2cl bezeichnen somit jeweils die linke Messelektrode und die Bezugszeichen 1cr und 2cr bezeichnen jeweils die rechte Messelektrode.

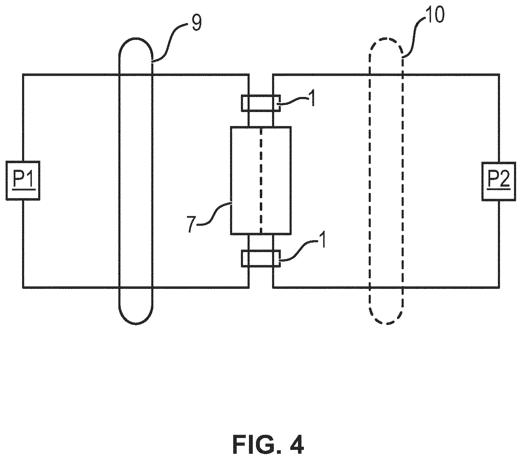

Fig. 4 ein Schaubild, welches den Einsatz eines erfindungsgemäßen Sensors zur Blut-Blut-Bilanzierung verdeutlicht.

In dem in Fig. 1 gezeigten Aufbau wird die Dialysefähigkeit einer gesunden Niere einer Person P2 einer Person mit Niereninsuffizienz P1 zur Verfügung gestellt. Dabei kommt ein Dialysator 7 mit einer Membran 8 zum Einsatz.

Ein erfindungsgemäßer Bilanzsensor kann verschiedentlich eingesetzt werden, um die Änderung des Flüssigkeitshaushalts bzw. Wasserbilanz der Person mit Niereninsuffizienz P1 zu messen.

Beispielsweise wird eine Bilanz (Eingang minus Ausgang des Dialysators) für die niereninsuffiziente Person P1 mittels eines erfindungsgemäßen Flusssensors 1 ermittelt. Diese Bilanz ist in Fig. 4 als Ellipse 9 angedeutet. Hierbei wird angenommen, dass die gesunde Person P2 ihren Wasserhaushalt selbstständig reguliert. Die Bilanz (Eingang minus Ausgang des Dialysators) für die gesunde Person P2 ist daher in Fig. 4 als gestrichelte Ellipse 10 dargestellt.

Die Bilanz für die gesunde Person kann mittels eines zweiten Flusssensors 1 ermittelt werden, der an der der gesunden Person P2 zugeordneten Seite des Dialysators Eingang gegen Ausgang bilanziert.

In Fig. 4 sind zwei erfindungsgemäße Sensoren 1 gezeigt. Dies ist jedoch lediglich ein Beispiel und es kann auch nur ein Flusssensor 1 vorgesehen sein, welcher die Fluidbilanz der Person P1 oder der Person P2 ermittelt.

## Patentansprüche

1. Flusssensor (1) zum Messen einer Flussdifferenz, mit
mindestens zwei Messkammern (2), durch welche Fluid leitbar ist;
mindestens einem Mittel zur Erzeugung eines Magnetfelds zur Ladungstrennung in einem die mindestens zwei Messkammern (2) durchströmenden Fluid, und
mindestens einem Mittel zur Messung eines elektrischen Potentials in dem die mindestens zwei Messkammern durchströmenden Fluid, wobei
die mindestens zwei Messkammern (2) derart angeordnet sind, dass sie von derselben Magnetfeldlinie des Magnetfelds zur Ladungstrennung durchlaufen werden **dadurch gekennzeichnet, dass** der Flusssensor (1) zusätzlich einen Temperatursensor zur Messung der Temperatur eines Fluids aufweist.

2. Flusssensor nach Anspruch 1, **dadurch gekennzeichnet, dass** der Flusssensor (1) ein magnetisch-induktiver differentieller Durchflusssensor ist.

3. Flusssensor (1) nach einem der vorhergehenden Ansprüche, **dadurch gekennzeichnet, dass** der Flusssensor (1) zusätzlich einen Leitfähigkeitssensor zur Messung der Leitfähigkeit eines Fluids aufweist.

4. Flusssensor (1) nach einem der vorhergehenden Ansprüche, **dadurch gekennzeichnet, dass** jeder der zwei Messkammern (2) ein Mittel zur Messung eines elektrischen Potentials zugeordnet ist und die mindestens zwei Messkammern (2) und / oder die diesen zugeordneten Mittel zur Messung eines elektrischen Potentials miteinander fluchten.

5. Flusssensor (1) nach einem der vorhergehenden Ansprüche, **dadurch gekennzeichnet, dass** der Flusssensor (1) als Disposable ausgestaltet ist.

6. Blutbehandlungsgerät, insbesondere Dialysegerät, mit einem Flusssensor (1) gemäß einem der Ansprüche 1 bis 5.

7. Blutbehandlungsgerät, insbesondere Dialysegerät, gemäß Anspruch 6, **dadurch gekennzeichnet, dass** das Blutbehandlungsgerät dazu ausgelegt ist, einen Fluss-sensor (1) gemäß einem der Ansprüche 1 bis 5 zumindest teilweise lösbar aufzunehmen und vorzugsweise zu diesem Zweck mit einer Aufnahme für den Fluss-sensor ausgestattet ist.

8. System mit einem Blutbehandlungsgerät und einem Flusssensor (1) gemäß einem der Ansprüche 1 bis 5, **dadurch gekennzeichnet, dass** das mindestens eine Mittel zur Erzeugung eines Magnetfelds zur Ladungstrennung in einem die mindestens zwei Messkammern (2) durchströmenden Fluid seitens des Blutbehandlungsgeräts anordnet ist.

9. System gemäß Anspruch 8, **dadurch gekennzeichnet, dass** das mindestens eine Mittel zur Erzeugung eines Magnetfelds zur Ladungstrennung mindestens zwei Magnetpole umfasst, welche auf einander gegenüberliegenden Seiten einer Aufnahme für den Flusssensor (1) angeordnet sind.

10. Verwendung eines Flusssensors (1) gemäß einem der Ansprüche 1 bis 6 und / oder eines Blutbehandlungsgeräts nach einem der Ansprüche 6 bis 7 zur Blut-Blut-Bilanzierung.

11. Verfahren zur Messung einer Flussdifferenz eines Fluids, vorzugsweise mit einem Flusssensor (1) gemäß einem der Ansprüche 1 bis 6, mit den Schritten:
Leiten von Fluid durch mindestens zwei Messkammern (2),
Erzeugen eines Magnetfelds zur Ladungstrennung in dem Fluid,
Messen eines elektrischen Potentials in dem die mindestens zwei Messkammern (2) durchströmenden Fluid, wobei
das Magnetfeld zur Ladungstrennung derart erzeugt wird, dass dieselbe Magnetfeldlinie des Magnetfelds zur Ladungstrennung die mindestens zwei Messkammern durchläuft, weiterhin mit dem Schritt:
Erfassen der Temperatur des Fluids, vorzugsweise mittels eines in den Flusssensor integrierten Temperatursensors.

12. Verfahren nach Anspruch 11, weiterhin mit dem Schritt:
Erfassen der Leitfähigkeit des Fluids, vorzugsweise mittels eines in den Fluss-sensor integrierten Leitfähigkeitssensors.

## Claims

1. A flow sensor (1) for measuring a flow difference, comprising
at least two measurement chambers (2) through which fluid is conductible;
at least one means for producing a magnetic field for charge separation in a fluid flowing through the at least two measurement chambers (2); and
at least one means for measuring an electric potential in the fluid flowing through the at least two measurement chambers, wherein
the at least two measurement chambers (2) are arranged such that they are run-through by the same magnetic field line of the magnetic field for charge separation, **characterized in that** the flow sensor (1) additionally has a temperature sensor for measuring the temperature of a fluid.

2. A flow sensor in accordance with claim 1, **characterized in that** the flow sensor (1) is a magnetically inductive differential flow sensor.

3. A flow sensor (1) in accordance with one of the preceding claims, **characterized in that** the flow sensor (1) additionally has a conductivity sensor for measuring the conductivity of a fluid.

4. A flow sensor (1) in accordance with one of the preceding claims, **characterized in that** each of the two measurement chambers (2) has a means for measuring an electric potential associated with it and the at least two measurement chambers (2) and/or the means for measuring an electric potential associated with them are aligned with one another.

5. A flow sensor (1) in accordance with one of the preceding claims, **characterized in that** the flow sensor (1) is designed as disposable.

6. A blood treatment device, in particular a dialysis machine, having a flow sensor (1) in accordance with one of the claims 1 to 5.

7. A blood treatment device, in particular a dialysis device in accordance with claim 6, **characterized in that** the blood treatment device is adapted to at least partly releasably receive a flow sensor (1) in accordance with one of the claims 1 to 5 and is preferably equipped with a mount for the flow sensor for this purpose.

8. A system having a blood treatment device and a flow sensor (1) in accordance with one of the claims 1 to 5, **characterized in that** the at least one means for producing a magnetic field for charge separation is arranged at the side of the blood treatment device in a fluid flowing through the at least two measurement chambers (2).

9. A system in accordance with claim 8, **characterized in that** the at least one means for producing a magnetic field for charge separation comprises at least two magnetic poles that are arranged at mutually opposite sides of a mount for the flow sensor (1).

10. Use of a flow sensor(1) in accordance with one of the claims 1 to 6 and/or of a blood treatment device in accordance with one of the claims 6 to 7 for blood-blood balancing.

11. A method of measuring a flow difference of a fluid, preferably using a flow sensor (1) in accordance with one of the claims 1 to 6, comprising the steps:
conducting fluid through at least two measurement chambers (2);
producing a magnetic field for charge separation in the fluid; and
measuring an electric potential in the fluid flowing through the at least two measurement chambers (2), wherein
the magnetic field for charge separation is produced such that the same magnetic field line of the magnetic field for charge separation runs through the at least two measurement chambers, further comprising the step:
detecting the temperature of the fluid, preferably by means of a temperature sensor integrated in the flow sensor.

12. A method in accordance with claim 11, further comprising the step:
detecting the conductivity of the fluid, preferably by means of a conductivity sensor integrated in the flow sensor.

## Revendications

1. Capteur de flux (1) pour mesurer une différence de flux, avec
au moins deux chambres de mesure (2) à travers lesquelles un fluide peut être acheminé ;
au moins un moyen pour générer un champ magnétique pour la séparation des charges dans un fluide traversant les au moins deux chambres de mesure (2), et
au moins un moyen pour mesurer un potentiel électrique dans le fluide traversant les au moins deux chambres de mesure,
les au moins deux chambres de mesure (2) étant agencées de telle sorte qu'elles sont parcourues par la même ligne de champ magnétique du champ magnétique pour la séparation des charges, **caractérisé en ce que** le capteur de flux (1) présente en outre un capteur de température pour mesurer la température d'un fluide.

2. Capteur de flux selon la revendication 1, **caractérisé en ce que** le capteur de flux (1) est un capteur de flux différentiel magnéto-inductif.

3. Capteur de flux (1) selon l'une quelconque des revendications précédentes, **caractérisé en ce que** le capteur de flux (1) présente en outre un capteur de conductivité pour mesurer la conductivité d'un fluide.

4. Capteur de flux (1) selon l'une quelconque des revendications précédentes, **caractérisé en ce qu'**un moyen de mesure d'un potentiel électrique est associé à chacune des deux chambres de mesure (2) et les au moins deux chambres de mesure (2) et/ou les moyens de mesure d'un potentiel électrique qui leur sont associés sont alignés les uns avec les autres.

5. Capteur de flux (1) selon l'une quelconque des revendications précédentes, **caractérisé en ce que** le capteur de flux (1) est réalisé sous forme de dispositif jetable.

6. Appareil de traitement du sang, notamment appareil de dialyse, avec un capteur de flux (1) selon l'une quelconque des revendications 1 à 5.

7. Appareil de traitement du sang, notamment appareil de dialyse, selon la revendication 6, **caractérisé en ce que** l'appareil de traitement du sang est conçu pour recevoir de manière au moins partiellement amovible un capteur de flux (1) selon l'une quelconque des revendications 1 à 5 et est de préférence équipé à cet effet d'un logement pour le capteur de flux.

8. Système avec un appareil de traitement du sang et un capteur de flux (1) selon l'une quelconque des revendications 1 à 5, **caractérisé en ce que** l'au moins un moyen de génération d'un champ magnétique pour la séparation des charges est agencé dans un fluide traversant les au moins deux chambres de mesure (2) du côté de l'appareil de traitement du sang.

9. Système selon la revendication 8, **caractérisé en ce que** l'au moins un moyen de génération d'un champ magnétique pour la séparation des charges comprend au moins deux pôles magnétiques agencés sur des côtés opposés d'un logement pour le capteur de flux (1).

10. Utilisation d'un capteur de flux (1) selon l'une quelconque des revendications 1 à 6 et/ou d'un appareil de traitement du sang selon l'une quelconque des revendications 6 à 7 pour l'équilibrage sang-sang.

11. Procédé de mesure d'une différence de flux d'un fluide, de préférence avec un capteur de flux (1) selon l'une quelconque des revendications 1 à 6, avec les étapes suivantes :
l'acheminement d'un fluide à travers au moins deux chambres de mesure (2),
la génération d'un champ magnétique pour la séparation des charges dans le fluide,
la mesure d'un potentiel électrique dans le fluide traversant les au moins deux chambres de mesure (2),
le champ magnétique pour la séparation des charges étant généré de telle sorte que la même ligne de champ magnétique du champ magnétique pour la séparation des charges parcoure les au moins deux chambres de mesure, avec en outre l'étape suivante :
la détection de la température du fluide, de préférence au moyen d'un capteur de température intégré dans le capteur de flux.

12. Procédé selon la revendication 11, avec en outre l'étape suivante :
la détection de la conductivité du fluide, de préférence au moyen d'un capteur de conductivité intégré dans le capteur de flux.
